Europäisches Patentamt

European Patent Office

Office européen des brevets

(11) Numéro de publication: **0 068 928**
**B1**

(12) FASCICULE DE BREVET EUROPEEN

(45) Date de publication du fascicule du brevet: **01.04.87**

(51) Int. Cl.⁴: **A 61 B 6/06, G 21 K 1/02**

(21) Numéro de dépôt: **82400985.6**

(22) Date de dépôt: **27.05.82**

(54) **Table d'examen radiologique comportant un sélecteur.**

(30) Priorité: **05.06.81 FR 8111222**

(43) Date de publication de la demande:
**05.01.83 Bulletin 83/01**

(45) Mention de la délivrance du brevet:
**01.04.87 Bulletin 87/14**

(84) Etats contractants désignés:
**DE IT NL**

(56) Documents cités:
**US-A-2 026 907**
**US-A-3 465 144**

(73) Titulaire: **THOMSON-CSF**
**173, Boulevard Haussmann**
**F-75379 Paris Cedex 08 (FR)**

(72) Inventeur: **Le Sonn, Marcel**
**THOMSON-CSF SCPI 173, bld Haussmann**
**F-75379 Paris Cedex 08 (FR)**

(74) Mandataire: **Grynwald, Albert et al**
**THOMSON-CSF SCPI 19, avenue de Messine**
**F-75008 Paris (FR)**

Courier Press, Leamington Spa, England.

**Description**

La présente invention concerne une table d'examen radiologique comportant un sélecteur, destinée au domaine du radio diagnostic.

Les différents statifs de radiologie, et tout particulièrement les statifs conventionnels à table télécommandée, comportent un sélecteur. Celui-ci, situé dans la table d'examen sous le panneau porte-patient est destiné à recevoir une cassette contenant un film radiographique, afin d'effectuer des radiogrammes selon un format désiré. A cet effet, généralement, la cassette se déplace dans le sélecteur sous un dispositif de masquage réglable, tel que par exemple des volets de masquage mobiles: les déplacements de cette cassette et de ces volets de masquage sont gérés par des moyens électro-mécaniques ou électroniques que comporte ce sélecteur, afin d'amener la cassette à occuper une position dans laquelle la surface de film radiographique exposée, correspond au format voulu. Une telle organisation apparaît dans un brevet US—A—3 465 144, qui décrit une cassette radiographique partagée en quatre cadrans utilisés successivement.

Il est fréquent de procéder à un examen radioscopique avant de fixer l'image radiologique virtuelle sur le film radiographique, et un détecteur de rayonnement tel qu'un amplificateur de luminance, est alors utilisé, comme décrit dans le brevet US ci-dessus mentionné. Ce brevet décrit une table d'examen dans laquelle un amplificateur de luminance est capable d'un déplacement à l'intérieur de la table d'examen selon un axe parallèle à l'axe longitudinal de cette dernière et, grâce à un dispositif d'asservissement, la position d'un sériographe peut être centrée sur celle de l'amplificateur de luminance; le seriographe étant maintenu à l'une ou l'autre des extrémités de la table en dehors des phases de radiographie. L'inconvénient de cette méthode est qu'elle augmente le temps mort entre radioscopie et radiographie du fait du transport du sériographe.

Dans d'autres cas, un sélecteur contenant une cassette radiographique est déplacé en même temps que l'amplificateur de luminance. Ceci permet, après la radioscopie, de procéder à la radiographie avec un temps mort minimum, le sélecteur étant déjà dans la bonne position: il faut bien entendu que durant la radioscopie, le passage du faisceau de rayonnement soit libéré, notamment en écartant les volets de de masquage et la cassette.

Aussi, existe-t-il des sélecteurs où la cassette radiographique, durant la séquence de radioscopie, est placée à l'abri du rayonnement X dans une position d'attente, dans un espace supplémentaire que comporte ce sélecteur; mais, ceci nécessite une dimension plus importante de ce dernier, qui doit alors comporter au moins une surface double de celle de la cassette radiographique. Cet accroissement de la dimension du sélecteur est réalisé, soit selon l'axe longitudinal de la table d'examen, soit selon l'axe transversal de celle-ci.

Les inconvénients en sont les suivants: en ce qui concerne l'accroissement de la dimension du sélecteur selon un axe longitudinal de la table d'examen: l'inconvénient consiste en ce que la partie active du sélecteur, c'est-à-dire celle dans laquelle le film radiographique est sensibilisée par le rayonnement X, ne peut être amenée jusqu'à l'une des extrémités de la table d'examen, à cause de l'encombrement présenté par cet espace supplémentaire. Ce défaut est important, en ce qu'il ne permet pas dans certains cas la poursuite aisée d'un examen, et peut nécessiter le déplacement du patient. Dans le cas où l'accroissement de la dimension du sélecteur s'effectue selon un axe transversal à la table d'examen: l'inconvénient précédemment cité n'existe plus; par contre, cette augmentation de la dimension du sélecteur conduit à augmenter dans les même proportions la largeur de la table d'examen.

La présente invention concerne une table d'examen radiologique, comportant un sélecteur, qui pour toutes les positions qu'il peut occuper dans la table d'examen, y compris les positions extrêmes, est opérationnel pour la radiographie, sans nuire aux caractéristiques habituelles telles que, protection de la cassette radiographique pendant la séquence de radioscopie, rapidité d'exécution et transparence au rayonnement X pour la radioscopie. Ceci est obtenu grâce à un agencement tout à fait nouveau de ce sélecteur, ne conduisant à aucune modification des dimensions habituelles d'une table d'examen.

Plus précisément, l'invention concerne une table d'examen radiologique permettant un examen radioscopique et radiographique comportant: un panneau porte-patient, un sélecteur et un amplificateur de luminance; lesdits sélecteur et amplificateur de luminance étant déplaçables sous le panneau porte-patent sur toute la longueur de ladite table; ledit sélecteur comportant un premier plateau porte-cassette supportant une cassette radiologique associée à des premiers moyens moteurs permettant des déplacements dudit premier plateau selon des axes transversaux par rapport à la table et un second plateau supportant le premier plateau porte-cassette associé à des seconds moyens moteurs permettant des déplacements dudit second plateau selon des axes longitudinaux par rapport à la table; lesdits premier plateau et respectivement second plateau et ladite cassette radiologique étant en position d'examen radiologique contenus dans un châssis, est caractérisé en ce que ledit châssis présente des ouvertures aménagées dans un premier et un second côtés qui sont transversaux par rapport à ladite table d'examen; en ce que lesdites ouvertures communiquent avec des zones de protection qui comportent des moyens de protection de la cassette radiographique contre un rayonnement X; et en ce que l'amplificateur de luminance et le châssis restant mutuellement centrés l'un par rapport à l'autre en position d'examen radioscopique et en position d'examen radiologique, les seconds moyens moteurs, en position d'examen radioscopique, transportent le second plateau au dehors du châssis au travers de l'ouverture aménagée dans le côté du châssis qui est le plus éloigné du bord transversal extrême de la table radiologique pour placer la cassette radiologique dans une zone de protection puis, en position d'examen radiologique réintroduisent le second plateau dans le châssis afin de replacer la cassette radiographique en position pour l'examen radiographique.

L'invention sera mieux comprise à l'aide des explications qui vont suivre données à titre d'exemple, et qui s'appuient sur une figure unique jointe.

La figure représente par une vue en perspective, une table d'examen de radiologique comportant un sélecteur selon l'invention.

La figure montre une table 1 d'examen d'un statif de radiologie, équipée d'un sélecteur 2 selon l'invention, dont le corps principal est un châssis placé immédiatement sous un panneau portepatient 3. Sous le sélecteur 2, un amplificateur de luminance 6, comporte des moyens assurant son déplacement manuel ou motorisé le long des barres 4 et 5; ce déplacement s'effectue dans tout l'espace intérieur de la table 1 selon un axe parallèle à l'axe x—x, lui-même parallèle à l'axe longitudinal de cette table. Dans l'exemple non limitatif de cette description, l'amplificateur de luminance 6 et le sélecteur 2, sont mécaniquement liés pour ce déplacement; ceci permet au sélecteur 2 d'être centré au-dessus de l'amplificateur de luminance 6, dans toutes les positions occupées par celui-ci d'une extrémité à l'autre de la table 1, afin de diminuer le temps nécessaire à passer de la radioscopie à la radiographie.

Dans une autre version, le sélecteur 2 comporte ses propres moyens de mouvement, et son positionnement au-dessus de l'amplificateur de luminance 6, est alors assuré par des moyens d'automatisme et d'asservissement que comporte le statif.

Une source de rayonnement X, non représentée, située au dessus de la table 1, est en fonctionnement associée alternativement à l'amplificateur de luminance 6 pour les séquences de radioscopie, et à une cassette radiographique 7 pour les séquences de radiographie; cette cassette radiographique, représentée sur la figure à l'extérieur du châssis 100, est contenue dans celui-ci durant les séquences de radiographie. La source de rayonnement X, est déplaçable au-dessus de la table 2 sur un axe parallèle à l'axe x—x; elle est positionnée, ainsi que l'amplificateur de luminance 6, soit par des commandes manuelles de l'opérateur, soit grâce aux circuits d'automatisme et d'asservissement du statif, auxquels l'opérateur peut se substituer à tout moment.

Pour la radioscopie, ces positionnements sont réalisés de manière à ce qu'un faisceau de rayonnement X, émis par cette source de rayonnement sous une incidence quelconque, selon un axe tel que par exemple l'axe z-z, soit centré sur l'amplificateur de luminance 6. Au préalable ce dernier, grâce à des moyens de levage qu'il comporte, et afin de conserver durant la radiographie les caractéristiques obtenues en radioscopie, est déplacé parallèlement à un axe $z_1$—$z_1$; ce déplacement de l'amplificateur de luminance 6, s'effectue de manière à ce que son plan de réception, non visible sur le dessin, occupe dans le sélecteur 2, un même niveau que celui occupé par le plan supérieur 37 de la cassette radiographique 7, quand elle est contenue dans le sélecteur 2. Durant la séquence de radioscopie, le sélecteur 1 ne doit présenter aucun obstacle au passage du faisceau de rayonnement X, et cette condition est réalisée de la manière suivante: d'une part, la cassette radiographique 7, normalement introduite par l'ouverture 8, dans le sélecteur 2 en début d'examen, est automatiquement transportée avant que ne débute l'émission du rayonnement X, à l'extérieur du châssis 100, où elle est abritée de ce rayonnement par une membrane souple 9; d'autre part, des volets de masquage 17, que comporte le sélecteur 2, capables d'un déplacement motorisé parallèlement à un axe y—y transversal à la table 1, sont écartés au maximum l'un de l'autre.

Le sélecteur 2 comporte des moyens coopérant avec les circuits d'automatisme du statif, qui pemettent de déterminer à tout moment, selon la position occupée par ce sélecteur dans la table 1, le côté par lequel doit être évacuée la cassette radiographique, ce côté étant celui opposé à l'extrémité de la table 1, dont le sélecteur 2 est le plus rapproché. Cette opération est excutée dès que le circuit d'automatisme perçoit l'ordre de passer en séquence de radioscopie, ou lorsque le sélecteur 2 est à proximité d'une extrémité de la table 1, du côté de laquelle se trouve déjà, hors de ce sélecteur, la cassette radiographique; dans ce dernier cas, l'automatisme prend en charge l'opération consistant à transporter la cassette radiographique sur le côté opposé du sélecteur 2. Ceci provoque évidemment une brève interruption de la vision radioscopique, susceptible de gêner l'examen; aussi, c'est généralement l'opérateur, qui connaissant le côté vers lequel doit se poursuivre l'examen et avant que celui-ci ne débute, commande lui-même ce transport de la cassette radiographique 7.

A cette fin, le châssis 100 est muni sur chacun de ses premier et second côtés 27 et 11 transversaux à la table 1, d'une ouverture 12 visible sur le dessin uniquement sur le premier côté 27, par laquelle peut passer un plateau 13 capable d'un déplacement motorisé sur les barres 4 et 5; ce plateau 13 supporte un plateau porte-cassette 14, supportant lui-même la cassette radiographique 7. Celle-ci est maintenue sur ce plateau portecassette 14 par des éléments 16 qui l'enserre et la maintienne dès son introduction dans le sélecteur 2; celui-ci comporte des capteurs coopérant avec les éléments 16, afin de permettre aux circuits d'automatisme de connaître la dimension de la cassette radiographique 7, ainsi que les positions relatives des volets 17, du plateau 13, et du plateau porte-cassette 14. Ce dernier est muni de moyens moteurs assurant son déplacement, se-

lon des axes transversaux à la table 1 et parallèles à l'axe y—y, dans des rainures 15 que comporte le plateau 13: le déplacement du plateau porte-cassette 14, permet le positionnement de la cassette radiographique 7, durant les séquences de radiographie.

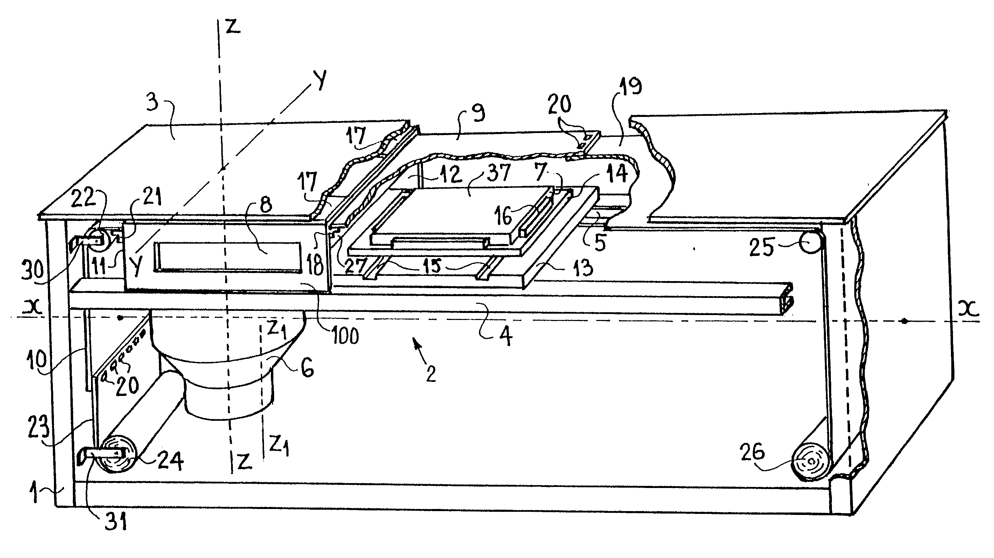

La figure 1 illustre particulièrement une situation fréquente où l'examen radioscopique est réalisé à une extrémité d'une table d'examen.

Dans l'exemple non limitatif de cette description, c'est sur le côté gauche de la table 1 qu'est effectué cet examen radioscopique et, l'amplificateur de luminance 6 ainsi que le sélecteur 2, sont centrées sur l'axe z—z du faisceau de rayonnement émis par la source située au-dessus de cette table; afin de ne pas créer d'obstables au passage du rayonnement X, les volets 17 du sélecteur 2 sont écartés et la cassette radiographique 7, grâce au déplacement du plateau 13 passant par l'ouverture 12, est transporté sur le côté droit du sélecteur 2, c'est-à-dire par le premier côté 27 du châssis 100, laissant le libre passage au faisceau de rayonnement X, tout en permettant au sélecteur 2 d'être en position pour la prochaine séquence de radiographie. Dans cette position, la cassette radiographique 7, est abritée du rayonnement X par un écran présentant une grande opacité au rayonnement X, et une souplesse mécanique lui permettant d'être courbé, tel que par exemple une membrane souple 9 constituée de caoutchouc à forte teneur en plomb. Cette membrane souple 9, fixée sur le premier côté 27 du châssis 100 par une équerre 18, est maintenue tendue par une toile 19 ordinaire dont elle est rendue solidaire par un moyen quelconque, comme par exemple des rivets 20. Cette toile est elle-même tendue par un dispositif dont sont visibles les extrémités des rouleaux 25 et 26 sur le côté droit du dessin, grâce à une ouverture pratiquée dans la paroi de la table 1, et dont l'équivalent, représenté sur le côté gauche de ce dessin, est explicité dans la suite de la description. Le second côté 11 du châssis 100 comporte une équerre 21 semblable à l'équerre 18, servant à fixer sur le sélecteur 2 une membrane souple 10, identique à la membrane souple 9, et dont la souplesse lui permet de tourner autour d'un rouleau 22, semblable au rouleau 25; ce rouleau 22 est fixé à la table 1 par des équerres 30, semblables aux équerres non représentées fixant le rouleau 25. L'extrémité de cette membrane souple est solidaire d'une toile ordinaire 23, de même nature que la toile 19, dont l'autre extrémité est fixée à un rouleau 24 identique au rouleau 26; ce rouleau 24 est fixé á la table 1, par des équerres 31 semblables aux équerres non représentées, fixant le rouleau 26. Grâce à un dispositif de ressort non représenté, le rouleau 24 exerce une traction constante sur le toile 23 tendant à l'enrouler quand le sélecteur 2 se rapproche de l'extrémité gauche de la table 1, et la laissant se dérouler quand le sélecteur s'en écarte, maintenant ainsi tendue la membrane souple 10. Cette description est également valable pour le dispositif servant à la tension de la membrane souple 9, étant bien entendu que le sens des actions est opposé.

Pour des positions plus centrales du sélecteur 2, les membranes souples 9 et 10 sont toutes deux horizontales; si le sélecteur 2 est situé à l'extrémité droite de la table 1, seule la membrane souple 10 est horizontale, prête à abriter la cassette radiographique qui durant la radioscopie est placée à l'extérieur gauche du sélecteur, par le déplacement du plateau 13.

Cette description montre que l'agencement d'un sélecteur selon l'invention, lui permet de rester centre au-dessus d'un amplificateur de luminance 6 servant à la radioscopie, pour toutes les positions que celui-ci est amené à occuper dans la table d'examen, y compris les extrémités, en maintenant une cassette radiographique 7 abritée du rayonnement X, sans nécessiter une dimension différente de la table d'examen pour la protection de cette cassette radiographique. Ceci est obtenu, grâce notamment à la coopération entre les membranes souples 9 et 10 avec respectivement les rouleaux 25 et 22, permettant le basculement d'un plan horizontal à un plan vertical, de celle de ces membranes qui arrive à une extrémité de la table 1, libérant ainsi la place nécessaire au positionnement du sélecteur 2 à cette extrémité.

A la fin de la séquence de radioscopie, l'amplificateur de luminance 6, libére l'espace qu'il occupe dans le sélecteur 2, et la cassette radiographique 7 grâce au déplacement du plateau 13, est réintroduite dans le sélecteur 2 où elle est prête à être utilisée. L'automatisme du sélecteur 2 combine les déplacements du plateau 13 et du plateau porte-cassette 14, avec le déplacement des volets 17, de manière à présenter au rayonnement X la surface de film à sensibiliser, selon le format désiré suivant un programme préétabli.

Le déplacement du plateau 13, vers le premier côté 27 ou vers le second côté 11 du sélecteur 2 grâce aux ouvertures 12 que celui-ci comporte, coopérant avec les membranes souples 9 et 10 permettent à un sélecteur selon l'invention, dans un premier temps, d'une part de libérer le passage du faisceau de rayonnement et d'autre part de placer une cassette radiographique 7 à l'abri de ce rayonnement X destiné à une radioscopie, et dans un deuxième temps, de réintroduire cette cassette radiographique dans le sélecteur 2 afin de procéder à la radiographie.

Un sélecteur selon l'invention peut équiper tout appareil de radiologie basculant ou non devant comporter un film radiographique, ou un autre composant sensible, susceptible d'être soumis à des alternances de sensibilisation et de protection, et ce pour une quelconque position dans l'espace d'une table qu'il équipe.

**Revendications**

1. Table d'examen radiologique permettant un examen radioscopique et radiographique comportant: un panneau porte-patient (3), un sélecteur (2) et un amplificateur de luminance (6);

lesdits sélecteur (2) et amplificateur de luminance (6) étant déplaçables sous le panneau porte-patient (3) sur toute la longueur de ladite table (1); ledit sélecteur (2) comportant un premier plateau (14) porte-cassette supportant une cassette radiologique (7) associé à des permiers moyens moteurs permettant des déplacements dudit premier plateau (14) selon des axes transversaux par rapport à la table (1) et un second plateau (13) supportant le premier plateau (14) porte-cassette associé à des seconds moyens moteurs permettant des déplacements dudit second plateau (13) selon des axes longitudinaux par rapport à la table; lesdits premier plateau (14) et respectivement second plateau (13) et ladite cassette radiologique (7) étant en position d'examen radiologique contenus dans un châssis (100), caractérisé en ce que ledit châssis (100) présente des ouvertures (12) aménagées dans un premier (27) et un second côtés (11) qui sont transversaux par rapport à ladite table (1) d'examen; en ce que lesdites ouvertures (12) communiquent avec des zones de protection qui comportent des moyens des protection (9, 10) de la cassette radiographique (7) contre un rayonnement X; et en ce que l'amplificateur de luminance (6) et le châssis (100) restant mutuellement centrés l'un par rapport à l'autre en position d'examen radioscopique et en position d'examen radiologique, les seconds moyens moteurs, en position d'examen radioscopique, transportent le second plateau (13) au dehors du châssis (100) au travers de l'ouverture (12) aménagéé dans le côté (27, 11) du châssis (110) qui est le plus éloigné du bord transversal extrême de la table (1) pour placer la cassette radiologique (7) dans une zone de protection puis, en position d'examen radiologique réintroduisent le second plateau (13) dans le châssis (100) afin de replacer la cassette radiographique (7) en position pour l'examen radiographique.

2. Table d'examen selon la revendication 1, caractérisé en ce que les moyens de protection contre un rayonnement X sont solidaires du sélecteur (2) sur ses côtés (27) et (11) où se trouvent les ouvertures (12).

3. Table d'examen selon l'une des revendications précédentes, caractérisé en ce que ces moyens de protection contre un rayonnement X sont constitués par des membranes souples (9) et (10).

4. Table d'examen selon la revendication 3, caractérisé en ce que les membranes souples (9) et (10), coopèrent chacune avec un rouleau respectivement (25) et (22) pour basculer d'un plan parallèle au plan dudit panneau porte-patient (3) à un plan perpendiculaire au plan de ce dernier.

5. Table d'examen selon l'une des revendications 3 et 4, caractérisé en ce que les membranes souples (9) et (10) comportent chacune un moyen coopérant avec un dispositif de tension pour les maintenir tendues.

6. Table d'examen selon la revendication 5, caractérisé en ce que les moyens que comportent les membranes souples (9) et (10) servant à les maintenir tendues, sont des toiles (19) et (23).

7. Table d'examen selon l'une des revendications 5 et 6, caractérisé en ce que les dispositifs de tension sont constitués par des rouleaux (26) et (24) autour desquels s'enroulent ou se déroulent les toiles (19) et (23).

8. Table d'examen selon l'une des revendications 3 à 7, caractérisé en ce que les membranes souples (9) et (10) sont constituées en caoutchouc à forte teneur en plomb.

**Patentansprüche**

1. Tisch für radioskopische und radiographische Röntgenuntersuchungen mit einer Patientenliege (3), einem Wähler (2) und einem Leuchtkraftverstärker (6), wobei die beiden letzteren unter der Patientenliege (3) über die ganze Länge des Tisches (1) verschiebbar sind und der Wähler (2) eine erste Platte (14), die eine Röntgenfilmkassette (7) trägt und ersten Motormitteln zugeordnet ist, die Verschiebungen der ersten Platte (14) gemäß bezüglich des Tisches (1) transversalen Achsen erlauben, und eine zweite Platte (13) aufweist, die die erste Platte (14) trägt und zweiten Motormitteln zugeordnet ist, die Verschiebungen dieser zweiten Platte (13) gemäß bezüglich des Tisches longitudinalen Achsen erlauben, und wobei die erste Platte (14), die zweite Platte (13) und die Röntgenfilmkassette (7) während der radiologischen Untersuchung in einem Chassis (100) enthalten sind, dadurch gekennzeichnet, daß in einer ersten (27) und einer zweiten Seitenfläche (11) des Chassis (100), die quer zum Untersuchungstisch (1) liegen, Öffnungen (12) vorgesehen sind, die mit Schnitzzonen in Verbindung stehen, wobei diese Schutzzonen Schnutzmittel (9, 10) für die radiographische Kassette (7) gegen eine Röntgenstrahlung aufweisen, und daß, während der Leuchtkraftverstärker (6) und das Chassis (100) während der radioskopischen und der radiologischen Untersuchungen gegenseitig zentriert bleiben, die zweiten Motormittel während der radioskopischen Untersuchungen die zweite Platte (13) aus dem Chassis (100) durch die Öffnung (12) herausbefördern, die in der von der äußersten Querwand des Tisches (1) am weitesten entfernten Seitenfläche (27, 11) des Chassis (100) angebracht ist, um die radiologische Kassette (7) in eine Schutzzone zu bringen, und daß dann die zweiten Motormittel für die radiographische Untersuchung die zweite Platte (13) wieder in das Chassis (100) einführen, um die radiographische Kassette (7) für die radiographische Untersuchung wieder in Stellung zu bringen.

2. Untersuchungstisch nach Anspruch 1, dadurch gekennzeichnet, daß die Schutzmittel gegen eine Röntgenstrahlung mit dem Wähler (2) an den Seiten (27 und 11) fest verbunden sind, an denen sich die Öffnungen (12) befinden.

3. Untersuchungstisch nach einem der vorhergehenden Amsprüche, dadurch gekennzeichnet, daß diese Schutzmittel gegen eine Röntgenstrahlung von nachgiebigen Membranen (9 und 10) gebildet werden.

4. Untersuchungstisch nach Anspruch 3, dadurch gekennzeichnet, daß die nachgiebigen Membranen (9 und 10) je mit einer Rolle (25 bzw. 22) zusammenwirken, um aus einer zur Ebene der Patientenliege (3) parallelen Ebene in eine dazu senkrechte Ebene zu kippen.

5. Untersuchungstisch nach einem der Ansprüche 3 und 4, dadurch gekennzeichnet, daß die nachgiebigen Membranen (9 und 10) je eine Mittel aufweisen, das mit einer Spannvorrichtung zusammenwirkt, um die Membranen gespannt zu halten.

6. Untersuchungstisch nach Anspruch 5, dadurch gekennzeichnet, daß die Mittel, die dazu dienen, die nachgiebigen Membranen (9 und 10) gespannt zu halten, Gewebebänder (19 und 23) sind.

7. Untersuchungstisch nach einem der Ansprüche 5 und 6, dadurch gekennzeichnet, daß die Mittel zum Spannen der nachgiebigen Membranen aus Rollen (26 und 24) bestehen, um die die Gewebebänder (19 und 23) auf- und abgerollt werden.

8. Untersuchungstisch nach einem der Ansprüche 3 bis 7, dadurch gekennzeichnet, daß die nachgiebigen Membranen (9 und 10) aus Gummi mit einem hohen Bleianteil bestehen.

**Claims**

1. A table for radioscopic and radiographic X-ray examinations comprising a panel (3) for supporting the patient, a selector (2) and a luminence amplifier (6), said selector (2) and luminence amplifier (6) being displaceable under the patient supporting panel (3) along the entire length of the table (1), said selector (2) comprising a first plate (14) supporting a radiographic cartridge (7) and associated to first motor means for allowing said first plate (14) to be displaced according to transversal axes with respect to the table (1), and a second plate (13) supporting the first plate (14) and associated to second motor means for allowing said second plate (13) to be displaced according to longitudinal axes with respect to the table, said first plate (14) and second plate (13) respectively and said radiographic cartridge (7) being contained in a chassis (100) during the radiographic examination, characterized in that said chassis (100) presents openings (12) in a first (27) and a second side surfaces (11) which are transversal with respect to said examination table (1), that said openings (12) communicate with protection zones comprising means (9, 10) for protecting the radiographic cartridge (7) against an X-radiation, and that, the luminence amplifier (6) and the chassis (100) remaining mutually centered during the radioscopic examination and during the radiographic examination, the second motor means transfer, during the radioscopic examination, the second plate (13) out of the chassis (100) through the opening (12) provided in the side surface (27, 11) of the chassis (100) which is the remotest side surface with respect to the transversal end edge of the table (1), in order to place the radiographic cartridge (7) in a protection zone, and that then these second motor means re-insert the second plate (13) into the chassis (100) for the radiographic examination in order to replace the radiographic cartridge (7) in a position allowing the radiographic examination.

2. An examination table according to claim 1, characterized in that the means for protection against an X-radiation are fixed to the selector (2) at its side surfaces (27 and 11) in which the openings (12) are provided.

3. An examination table according to one of the preceding claims, characterized in that these means for protection against an X-radiation are constituted by resilient diaphragms (9 and 10).

4. An examination table according to claim 3, characterized in that the resilient diaphragm (9 and 10) each cooperate with a roller (25 and 22 respectively) for tilting the diaphragms from a plane parallel to the plane of said panel (3) bearing the patient to a plane which is perpendicular to the plane of the latter.

5. An examination table according to one of claims 3 and 4, characterized in that the resilient diaphragms (9 and 10) each cooperate with means cooperating with a device for maintaining them tensioned.

6. An examination table according to claim 5, characterized in that the means serving to maintain the resilient diaphragms (9, 10) tensioned are tissues (19 and 23).

7. An examination table according to one of claims 5 and 6, characterized in that the tensioning devices are constituted by rollers (26 and 24) on which the tissues (19 and 23) are wound or unwound.

8. An examination table according to one of claims 3 to 7, characterized in that the resilient diaphragms (9 and 10) are made of caoutchouc having a high lead content.